(19) Europäisches Patentamt
European Patent Office
Office européen des brevets

(11) **EP 2 042 078 B1**

(12) **EUROPÄISCHE PATENTSCHRIFT**

(45) Veröffentlichungstag und Bekanntmachung des Hinweises auf die Patenterteilung:
**03.04.2013 Patentblatt 2013/14**

(51) Int Cl.:
***A61B 3/11*** *(2006.01)*     ***A61B 3/113*** *(2006.01)*

(21) Anmeldenummer: **08022289.6**

(22) Anmeldetag: **16.02.2002**

(54) **Verfahren zur Ermittlung von Abständen am vorderen Augenabschnitt**

Method for determining distances from the front eye section

Procédé de détermination d'intervalles au niveau de la section avant de l'oeil

(84) Benannte Vertragsstaaten:
**AT BE CH CY DE DK ES FI FR GB GR IE IT LI LU MC NL PT SE TR**

(30) Priorität: **21.02.2001 DE 10108797**

(43) Veröffentlichungstag der Anmeldung:
**01.04.2009 Patentblatt 2009/14**

(62) Dokumentnummer(n) der früheren Anmeldung(en) nach Art. 76 EPÜ:
**02716785.7 / 1 303 210**

(73) Patentinhaber: **Carl Zeiss Meditec AG**
**07745 Jena (DE)**

(72) Erfinder:
• **Bergner, Roland**
**07745 Jena (DE)**

• **Barth, Roland**
**07743 Jena (DE)**
• **Doering, Axel**
**07745 Jena (DE)**
• **Behrendt, Frank**
**07743 Jena (DE)**
• **Voigt, Klaus-Ditmar**
**07745 Jena (DE)**

(74) Vertreter: **Geyer, Fehners & Partner**
**Patentanwälte**
**Perhamerstrasse 31**
**80687 München (DE)**

(56) Entgegenhaltungen:
| | |
|---|---|
| WO-A-00/33729 | DE-A- 19 618 883 |
| US-A- 5 231 674 | US-A- 5 327 191 |
| US-A- 5 474 548 | |

**Beschreibung**

Für die IOL(Intraokularlinse) -Berechnungsformel "Holladay 2" ("Intraocular Lens Power Calculations for the Refractive Surgeon", Jack T. Holladay, in: Operative Techniques in Cataract and Refractive Surgery, Vol. 1, No 3 (September), 1998: pp 105-117), mit welcher die Stärke einer in das menschliche Auge zu implantierenden Intraokularlinse (IOL) berechnet werden kann, sowie für die Auswahl spezieller IOL Typen (ICL u.a.) wird als ein Eingangsparameter der sogenannte "horizontale-white-to-white-Abstand" (hor-w-t-w) benötigt. Das ist der horizontale Durchmesser der Iris.

Für die Homhautchirurgie zur Beseitigung von Fehlsichtigkeiten des menschlichen Auges (PRK, LASIK) ist es für den Operateur auch von Interesse, an welchem Punkt die Sehachse des Patienten die Hornhaut durchstößt. Danach kann die Laserabtragung von diesem Punkt aus präziser erfolgen, als mit der bisherigen Annahme, welche vom geometrischen Mittelpunkt der Hornhaut ausgeht.

Für die interferometrische Längenmessung der Hornhautdicke, Vorderkammertiefe und Linsendicke am menschlichen Auge mittels PCI ist es notwendig, das Auge entlang seiner hypothetischen optischen Achse vor dem Meßgerät voreinzustellen, im Unterschied zur Achslängenmessung, bei welcher das Auge entlang der tatsächlichen Sehachse positioniert werden muß.

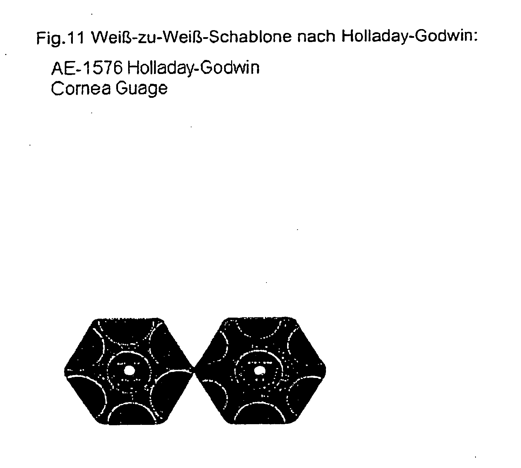

Zur Ermittlung des "hor-w-t-w" werden bisher Lineale bzw. Schablonen verwendet (Fig.11 und http://www.asico.com/1576.htm). welche dem Patientenauge vorgehalten werden und damit der Durchmesser der Iris durch Peilen abgelesen wird. Dieses Verfahren ist störanfällig gegenüber Parallaxe beim Beobachten und die bisher verwendeten Schablonen haben eine Abstufung von 0,5 mm. Damit ist nur eine eingeschränkte Genauigkeit möglich. Eine andere bekannte Lösung sind Meßokulare, welche an Spaltlampengeräten als Zubehör zum Einsatz kommen (Gebrauchsanweisung Spaltlampe 30 SUM Druckschriften Nr: G 30-114-d (MA XI/79) Carl Zeiss D-7082 Oberkochen, Seite 38). Diese verhindern zwar Parallaxefehler, es muß aber an einer Skale der Durchmesserwert abgelesen werden.

Weiterhin sind invasive Meßmittel in Form von mechanischen Meßschiebern bekannt, welche durch einen Schnitt in der Sklera in die Vorderkammer eingeführt werden (z.B. US 4319564).

Weiterhin sind sogenannte Gonioskope bekannt, welche auf das Auge aufgesetzt werden, Skalen auf die Iris projizieren und durch Lupen kann der Irisdurchmesser abgelesen werden (z.B. US 4398812).

Vorrichtungen, welche den Durchmesser der Pupille messen sind sogenannte Pupillometer (z.B. EP 0550673). Diese messen aber nicht den Durchmesser der Iris.

Für die Bestimmung des Durchtrittspunktes der Sehachse durch die Hornhaut sind keine Vorrichtungen bekannt. In der Kameraindustrie werden lediglich Verfahren verwendet, welche die Blickrichtung eines menschlichen Auges detektieren; die Ausgangssignale solcher Anordnungen dienen zum Beispiel der Steuerung von Autofokusmechanismen in Fotokameras (z.B. US 5291234) bzw. sie werden in sogenannten Eyetrackern eingesetzt. Diese Geräte überwachen die Augen- bzw. Blickbewegungen.

Zur Voreinstellung des Auges entlang der optischen Achse sind ebenfalls keine Vorrichtungen bekannt; es wird durch Umfixieren des Auges bzw. Scannen des Meßstrahles relativ ungezielt versucht, die richtige Positionierung des Auges entlang der optischen Achse durch Probieren zu finden.

Aus WO 00/33729 der Anmelderin ist eine Anordnung und ein Verfahren bekannt, um mittels eines einzigen Gerätes zur Berechnung der optischen Wirkung einer Introkularlinse berührungslos die Achslänge, Hornhautkrümmung und Vorderkammertiefe des Auges zu bestimmen.

Das Auge wird über sichtbare oder IR -LED-s allgemein beleuchtet, wobei deren Reflexbilder mittels der CCD Kamera erfaßt und dargestellt werden. Weiterhin ist ein Fixierlicht vorgesehen, damit der Proband die Augenpupille in Richtung der optischen Achse ausrichtet, wobei der Reflex des Fixierlichtes ebenfalls von der CCD - Kamera erfaßt wird.

Die DE 19618883 und die US 5231674 betreffen die Verwendung eines Fixierlichtreflexes zur Ermittlung des Durchstosspunktes der Sehachse durch die Hornhaut.

Aufgabe ist es, eine Vorrichtung und ein Verfahren zu schaffen, welches bedienerunabhängig eine höhere Genauigkeit der hor-w-t-w Bestimmung zuläßt .

Überraschend realisiert die Erfindung auch eine praktikable Möglichkeit den Durchtrittspunkt der Sehachse durch die Hornhaut in Bezug auf den Mittelpunkt der Pupille und / oder Iris zu beschreiben und aus der Position dieses Durchtrittspunktes und der geometrischen Mitte der Hornhaut eine präzisere Voreinstellung eines interferometrischen Längenmeßgerätes entlang der optischen Achse des Auges vornehmen zu können.

Bisher war die Lage der Sehachse dem Gerätebediener unbekannt; deshalb wurde der Patient mit geeigneten Mitteln (Fixierlicht im Gerät oder außerhalb des Gerätes für das Partnerauge) aufgefordert, umzufixieren. Im Anschluß wurde ein Meßvorgang ausgelöst, der aber nur erfolgreich ist, wenn die Messung entlang der optischen Achse erfolgte. Das bedeutet, daß erst im Anschluß an den Meßvorgang klar wird, ob diese Achse getroffen wurde oder nicht.

In Schritten von 1° wird das Fixierlicht bewegt; es können recht viele unbrauchbare Meßvorgänge erforderlich werden, bis die Stelle erreicht wird, wo die interferometrische Messung erfolgreich ist.

Für die ophthalmologische Routine ist diese Vorgehensweise nicht akzeptabel.

Ein Verfahren gemäß der Erfindung ist in Anspruch 1 definiert.

Ausführungsbeispiel:

Die Erfindung wird nachstehend anhand der schematischen Zeichnungen näher erläutert.

Es zeigen:

Fig.1: Den schematischen Ablauf des erfindungsgemäßen Verfahrens
Fig.2: Den Algorithmus zur Grobdetektion der Pupille
Fig.3: Eine Darstellung zur Grauwertanalyse / Mittelpunktsermittlung

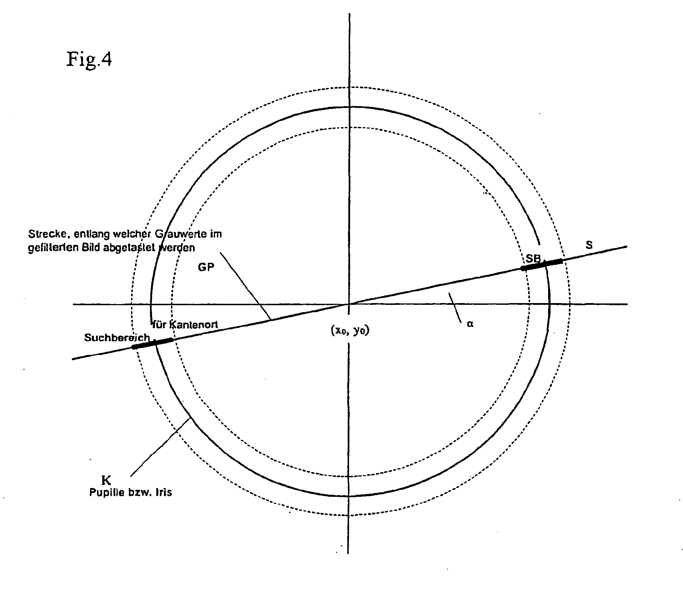

Fig.4: Eine Darstellung der Kantenanalyse
Fig.5: Den schematischen Ablauf der Kantenanalyse
Fig.6: Die Detektion und Ermittlung der Lage des Fixierpunktes
Fig.7: Den schematischen Ablauf der Plausibilitätskontrolle
Fig.8: Den Beleuchtungs/Detektionsstrahlengang
Fig.9: Ein Überblicksbild des zu vermessenden Auges
Fig.10: Die vergrößerte Darstellung des Zentrums in Fig.9
Fig.11: Eine Weiss zu Weiss Schablone nach Holladay-Godwin

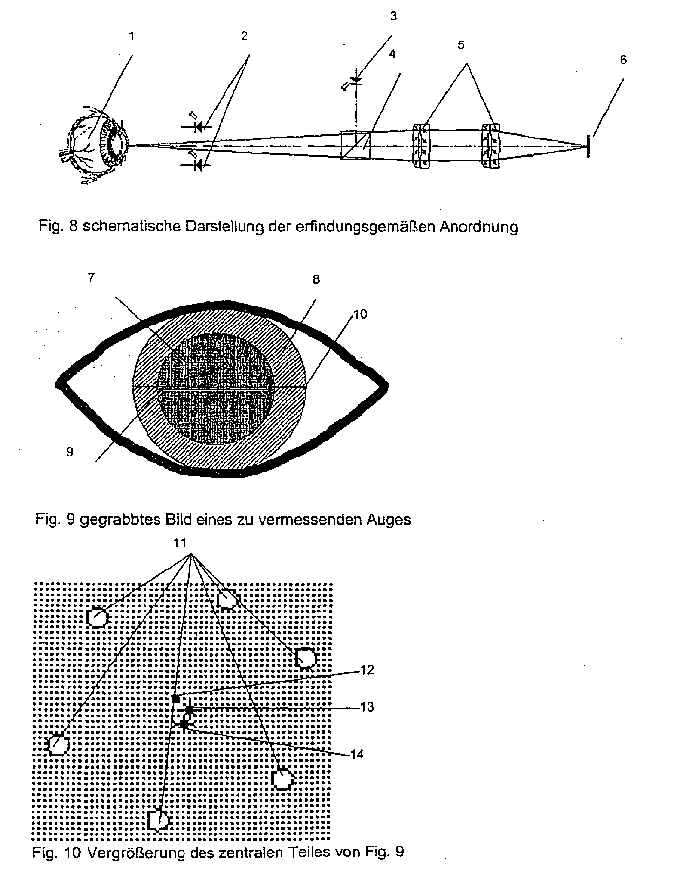

Das Probandenauge 1 wird gemäß Fig.8 durch vorzugsweise infrarot strahlende kreisförmig um die optische Achse angeordnete Lichtquellen 2, wie in WO 00/33729 (z.B. LED) beleuchtet. Im Beobachtungssystem wird koaxial zum Beobachtungsstrahlengang eine Lichtquelle 3 über einen Strahlteiler 4 eingeblendet, welche sichtbares Licht aussendet (z.B. LED oder Laserdiode), auf welche der Proband fixiert.
Das Bild des Auges wird über ein telezentrisches Abbildungssystem 5 auf einen Bildsensor 6,vorzugsweise eine CCD - Kamera, der mit einer nicht dargestellten Steuer-und Auswerteeinheit verbunden ist, abgebildet. Das Videosignal der Kamera wird auf einem ( nicht dargestellten) Monitor oder LC Display dargestellt.
Der Bediener kann sich durch die Beleuchtung 2 während der gesamten Zeit der Einjustierung und der Vermessung des Probanden davon überzeugen, daß der Proband richtig fixiert - und somit das Meßergebnis unverfälscht ist.
Die Abbildung des Probandenauges mit den relevanten Bildausschnitten wird telezentrisch durchgeführt, um den Einfluß der Probandenjustierung zu minimieren.
Das BAS - Signal der CCD - Kamera wird nach der korrekten Einjustierung des Patientenauges und nach Auslösung durch den Bediener mittels Frame Grabber in den Speicher eines Rechners in die Auswerteeinheit übernommen. In Fig.9 ist ein derartiges Bild schematisch dargestellt, mit Pupille 7, Pupillendurchmmesser 9 sowie Iris 8 und Irisdurchmesser 10.
Fig.10 zeigt einen vergrößerten Abschnitt der Pupille mit Reflexpunkten 11 der Beleuchtung, dem Bild des Fixierlichtes 12 , Irismittelpunkt 13 und Pupillenmittelpunkt 14.
Mit Mitteln der Bildverarbeitung werden die Abstände im Bild ermittelt, aus denen über den Abbildungsmaßstab der Beobachtungsoptik folgende Größen berechnet werden können:

- der Durchmesser und der Mittelpunkt der Iris,
- der Durchmesser und der Mittelpunkt der Pupille,
- die x- und y-Koordinaten des Hornhautbildes des Fixierlichtes (1. Purkinjebild) in Bezug auf den Mittelpunkt der Iris und
- die x- und y-Koordinaten des Hornhautbildes des Fixierlichtes ( 1.Purkinjebild) in Bezug auf den Mittelpunkt der Pupille

Da die realen Formen von Iris und Pupille des menschlichen Auges nicht notwendigerweise Kreise sein müssen, können in einer weiteren Ausführungsform auch Ellipsen mit ihren Parametern Halbachsen und Brennpunkte bestimmt werden.
Die Meßgrößen lassen sich bei geeigneter Wahl des Abbildungsmaßstabes der Abbildungsoptik 5 mit einer rechnerischen Genauigkeit von $< \pm\, 0,01$ mm bestimmen.
Der Durchmesser der Iris ergibt den horizontalen white-to-white Abstand: white-to-white [in mm] = $\varnothing_{iris}$ [in Pixeln] / Anzahl der Pixel pro mm
Die x-und y-Koordinaten des Purkinjebildes des Fixierlichtes ergeben den Durchstoßungspunkt der Sehachse durch die Hornhaut unter der Voraussetzung, daß der Proband richtig fixiert, was der Bediener anhand des Video-life-Bildes auf dem LC-Display während der Messung kontrollieren kann. Sehachse und opt. Achse können bis zu 8° voneinander abweichen, da die Fovea 3° nasal bis 8° temporal versetzt liegen kann.

( Vereinfachtes schematisches Auge nach Gullstrand in Diepes "Refraktionsbestimmung" Verlag Bode, Pfortzheim , 5. Auflage 1988).

Der Winkel zwischen der Sehachse und der optischen Achse des Auges ergibt sich aus Winkelbeziehungen beispielsweise anhand des Gullstrand- Auges, in welche die gemessene Ablage (Abstand) des Bildes des Fixierpunktes vom Iris- und/oder Pupillenmittelpunkt eingeht.

Vor der interferometrischen Vermessung der vorderen Augenmedien wird die Abweichung beider Sehachsen zueinander bestimmt.

Der Fixierpunkt des vorliegenden Meßsystems markiert sich entlang der Sehachse. Der Bertrag und die Richtung des Abstandes dieses Punktes zur Pupillenmitte (und oder Irismitte) wird ermittelt.

Durch einfache trigonometrische Formeln erhält man den gesuchten Winkel zwischen optischer Achse und Sehachse, beispielsweise zu

$$\alpha = \text{arc tan } (a / k)$$

$\alpha$ - Winkel zwischen Seh- und opt. Achse

a - Abstand Fixierpunkt zur Pupillenmitte (Irismitte)

k - Abstand Knotenpunkt (s. Literatur Diepes) zur Hornhaut minus R/2 (etwa 3,8 mm)

Entsprechend dieses Meßwertes kann vorteilhaft eine Voreinstellung der Blickrichtung des Patienten erfolgen, indem dem Patienten ein Fixierlicht unter dem errechneten Winkel $\alpha$ angeboten wird. Damit wird ein aufwendiges Suchverfahren überflüssig.

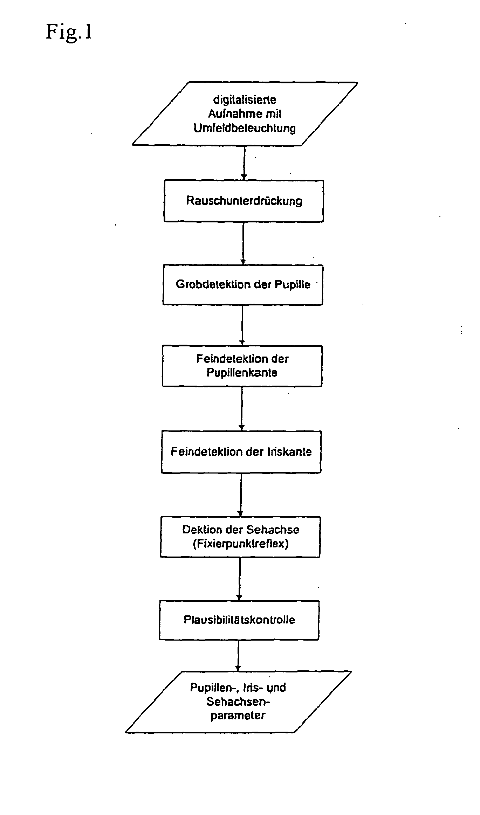

### Ablaufplan (Fig.1):

Als Eingabegröße der Auswertung wird eine digitalisierte Graubildaufnahme verwendet, in einem Abbildungsmaßstab, der eine Erfassung der gesamten Iris erlaubt, mit eingeschalteter Umfeldbeleuchtung.

In der Auswerteeinheit werden nach Rauschunterdrückung die Objekte Pupille, Iris und Fixierpunktbild bestimmt.

Vorteilhaft wird zunächst das Pupillenbild grob ermittelt und zur Irisdetektion herangezogen, da der Kontrast an der Iriskante in der Regel schwach ist und außerdem der Irisrand oben und unten durch Augenlider verdeckt sein kann, so daß keine kreisförmige sondern nur eine sektorförmige Erfassung möglich ist.

Bei erfolgreicher Abarbeitung werden die Parameter der Iris und der Pupille als Kreismodell (Radius, Mittelpunkt) oder als Ellipsenmodell (Hauptachsen, Mittelpunkt) zurückgegeben. Der Fixierpunkt (d.h. der Durchtrittspunkt der Sehachse durch die Kornea) wird in seinen Koordinaten zurückgegeben, d.h., die Koordinaten stehen dem aufrufenden Programm zur Verfügung.

### Rauschunterdrückung

Eine Kantendetektion auf der Basis von Grauwertprofilen im Originalbild führt zu großen Streuungen bei der Bestimmung der Kantenorte, die auf ein dem Bildsignal überlagertes Rauschen zurückzuführen sind. Zur Rauschunterdrückung wird ein 20x20-Medianfilter eingesetzt.

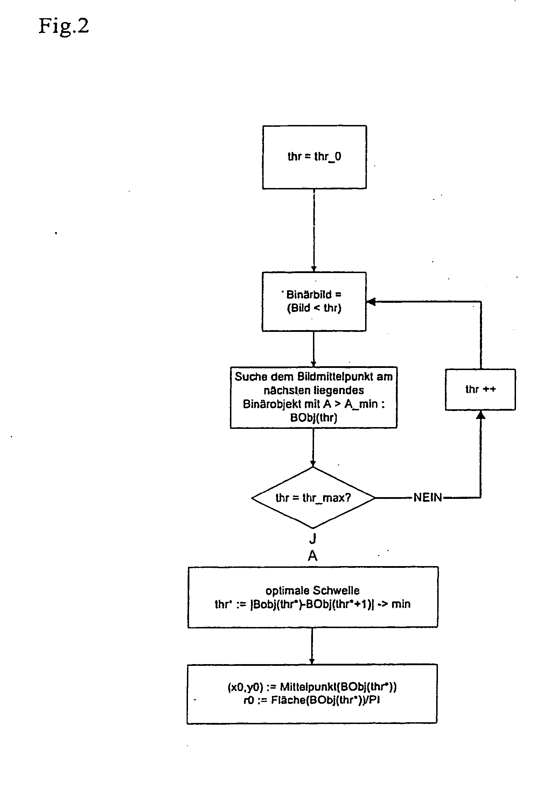

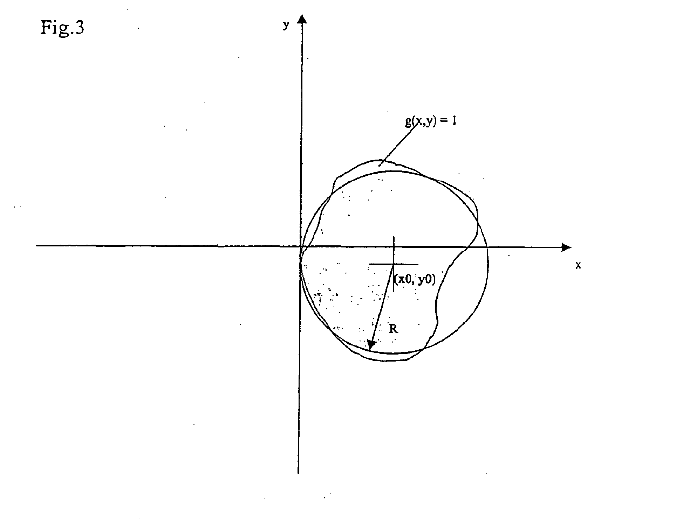

### Grobdetektion der Pupille- Fig.2/Fig.3

Zur groben Bestimmung der Pupillenlage wird ein Binarisierungsverfahren mit anschließender Suche nach zusammenhängenden Objekten im Binärbild verwendet.

In Fig.3 ist eine von der CCD Kamera erfaßte unregelmäßige Grauwertverteilung g(x,y), ermittelt durch Schwellwertanalyse (Schwellwert 1), in einem X/Y Koordinatensystem dargestellt. Von dieser Fläche wird über eine Schwerpunktanalyse der theoretische Mittelpunkt xo,yo bestimmt und ein Kreismodell/ Ellipsenmodell mit einem Radius R ermittelt. (Das wird weiter unten erklärt.)

Als Binarisierung wird die pixelweise Grauwerttransformation nach $b(x,y) = \begin{cases} 1 & wenn\ g(x,y) \geq thr \\ 0 & sonst \end{cases}$

verstanden, wobei gilt:

x        horizontale Koordinate eines Pixels

y        vertikale Koordinate eines Pixels
g(x,y)    Grauwert des Pixels an der Stelle (x,y)
thr      nicht-negativer Schwellwert

Als Pupille wird dasjenige Binärobjekt angenommen, das eine vorgegebene Mindestgröße überschreitet und dem Bildmittelpunkt am nächsten liegt. Ein Binarisierungsverfahren mit konstantem Schwellwert ist - wegen seiner Abhängigkeit von der Umgebungshelligkeit - nicht geeignet. Daher werden nach o.g. Verfahren Binärobjekte für eine Folge von Schwellwerten bestimmt. Als "optimaler" Schwellwert thr* wird derjenige Wert angenommen, bei dessen inkrementierung die geringste Veränderung beim ausgewählten Binärobjekt (d.h., Lage und Größe) stattfindet. Aus dem diesem Schwellwert zugeordneten Binärobjekt werden als Grobschätzung der Pupillenlage folgende Größen bestimmt:

$$x_0 = \frac{\sum g(x,y) \cdot x}{\sum g(x,y)}$$      (Summation über alle Pixel des Bildes)

$$y_0 = \frac{\sum g(x,y) \cdot y}{\sum g(x,y)}$$

$$R = \sqrt{\frac{1}{\pi} \sum g(x,y)}$$

wobei

$$g(x,y) = \begin{cases} 1 & (x,y) \in Bin\ddot{a}robjekt \\ 0 & sonst \end{cases}$$

$(x_0, y_0)$     Flächenschwerpunkt des Binärobjekts (Mittelpunktskoordinaten)
R         (Fläche des Binärobjekts / $\pi$)$^{1/2}$ (geschätzter Radius).

Die Kantenorte für Pupille und Iris (Kante = "Rand" des Kreises/der Ellipse) werden aus Grauwertprofilen (=Abtastungen der Grauwerte des medianfilterten Bildes entlang Strecken) durch den Mittelpunkt der grob bestimmten Pupille ermittelt (siehe Abbildung ). Für die Iris wird zunächst angenommen, daß sich die Kante in einem bestimmten größeren, konzentrisch zur grob detektierten Pupille angeordneten Kreisring befindet. Der folgende Algorithmus gilt sinngemäß sowohl für die Feindetektion der Pupillen- als auch der Iriskante.
Die Abtastung erfolgt mittels Suchstrahlen (Suchrichtungen) S von xo,yo aus, wobei die Suchstrahlrichung um einen Winkel $\alpha$ sukzessive geändert wird.
Der grobe Suchbereich SB auf dem Suchstrahl S ergibt sich durch das bereits ermittelte grobe Modell von Iris und Pupille. Die Bestimmung der Pupillenkante K erfolgt über den gesamten Kreis, während die Iriskantenbestimmung wegen möglicher Lidabdeckung nur in einem Winkelbereich um die X-Achse erfolgt (2 Kreissektoren).
In diesen Profilen werden Wendepunkte durch eine geeignete Glättung und numerische Differentiation bestimmt. Hierzu ist eine Reihe von Verfahren (z.B. Savitzky A. and Golay, M.J.E. Analytical Chemistry, Vol. 36, S. 1627-39, 1964) bekannt, die sich als eindimensionale lineare Filter effizient implementieren lassen. Im allgemeinen wird eine Vielzahl von Wendepunkten entlang des Grauwertprofils gefunden. Als Kantenort wird unter diesen diejenige Position (x,y) ermittelt, die folgende Bedingungen erfüllt:

(a) (x,y) liegt in einem Kreisring um $(x_0,y_0)$.(Grobposition der Pupille) mit einem inneren und äußeren Radius, der jeweils für die Pupillen- und Irisdetektion in Abhängigkeit von r0 (Grobradius der Pupille) bestimmt werden kann
(b) Die Differenz zwischen den um (x,y) im Grauwertprofil liegenden Extremwerten wird betragsmäßig maximal unter allen Positionen, die (a) erfüllen.

Damit stehen pro Grauwertprofil (d.h., pro Abtastwinkel $\alpha$) maximal je zwei Kantenorte für die Iris- und die Pupillenmodellierung zur Verfügung. Zur Ausschaltung systematischer Störungen beispielsweise durch Abdeckung der Iris oder der Pupille bei verengter Lidspalte kann der verwendete Bereich von Abtastwinkeln eingeschränkt werden, d.h. $\alpha_{min, Iris} < \alpha < \alpha_{max, Iris}$ für die Iriskantenbestimmung und analog $\alpha_{min, Pupille} < \alpha < \alpha_{max, Pupille}$ für die Pupillenkantenbestimmung.

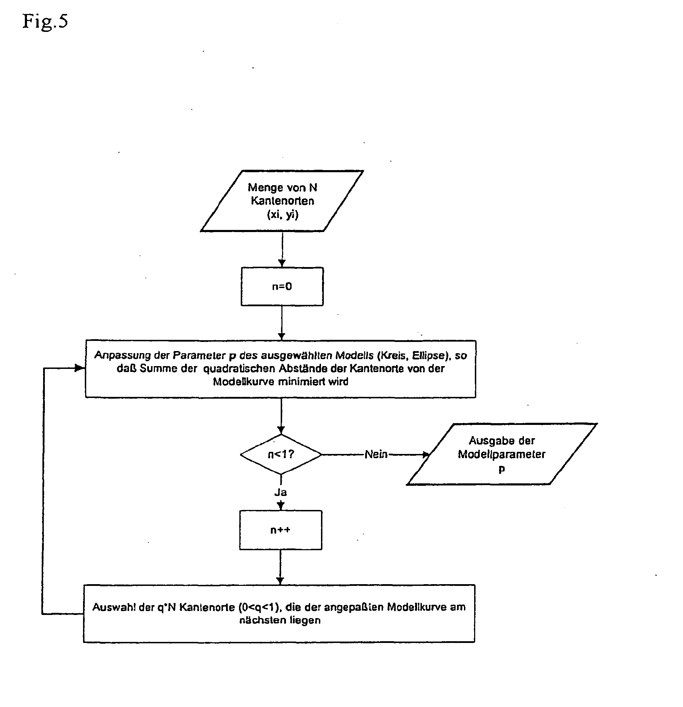

**Anpassung des Pupillen-/Irismodells - Fig.5**

Aus der Menge der im vorhergehenden Schritt bestimmten Kantenorte ($x_i$, yi) können die Parameter des anzupassenden Pupillen- und Irismodells (also entweder Kreis oder Ellipse) durch Regression bestimmt werden. Dies geschieht durch die Minimierung der Summe quadratischer Fehler

$$\Sigma_i \, (x_i - x(x_i, y_i, \mathbf{p}))^2 + (y_i - y(x_i, y_i, \mathbf{p}))^2 \rightarrow \min \qquad\qquad (1)$$

über die Menge möglicher Parametervektoren p (Kreis: Mittelpunktskoordinaten und Radius, Ellipse: Mittelpunktskoordinaten, Längen der Hauptachsen, Winkel der großen Hauptachse zur x-Achse). Für die Kreisanpassung ist eine Lösung von (1) unmittelbar und numerisch effizient mit dem Verfahren der Singulärwertzerlegung möglich. Für die Lösung des restringierten Quadratmittelproblems zur Ellipsenanpassung existiert ebenfalls eine Reihe von Standardansätzen (z.B. in Bookstein, F.L. Fitting conic sections to scattered data. Computer Graphics and Image Processing, Vol. 9, S. 56-71, 1979 und Fitzgibbon, A.W. and Fisher, R.B. A buyer's guide to conic fitting. Proceedings of British Machine Vision Conference, Birmingham, 1995).

Zur Reduktion des Einflusses von Ausreißern (d.h., falsch bestimmten Kantenorten) wird ein zweistufiges Regressionsverfahren nach Fig.5 verwendet.

Außerdem können alternative Verfahren zur Selektion der Kantenorte, die zur Parameteranpassung verwendet werden sollen, genutzt werden, wie beispielsweise die Hough-Transformation für Kreismodelle.

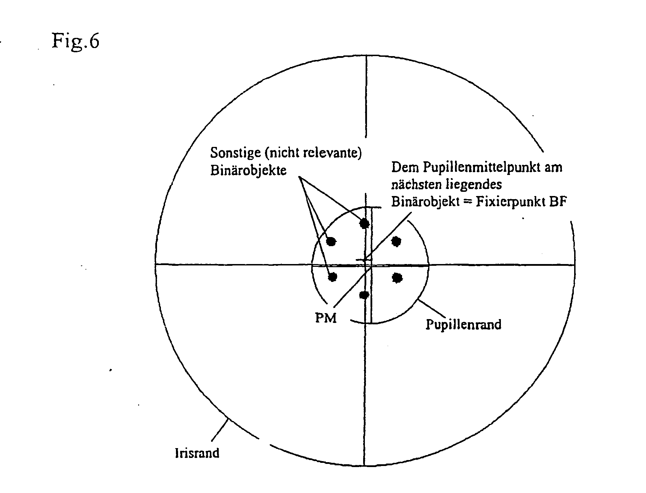

**Detektion des Fixierpunktes- Fig.6**

Zur Detektion des Fixierpunktes erfolgt eine Binarisierung ( siehe oben) des ungefilterten Bildes der CCD Kamera mit einem von thr* (Schwellwert, der zur Grobdetektion der Pupille benutzt wurde) abhängigen Schwellwert $\Theta_{FP}$ = s*thr* (s>1.0). Als Fixierpunkt wird der Mittelpunkt des dem bestimmten Pupillenmittelpunkt PM am nächsten liegenden zusammenhängenden Binärobjektes BF (Grauwerte > $\Theta_{FP}$) bestimmt, das eine bestimmte Mindestfläche aufweist. Sonstige nicht relevante Binärobjekte (z.B. Reflexbilder der LED Beleuchtung) werden anhand ihrer größeren Entfernung vom Pupillenmittelpunkt identifiziert und bleiben unberücksichtigt.

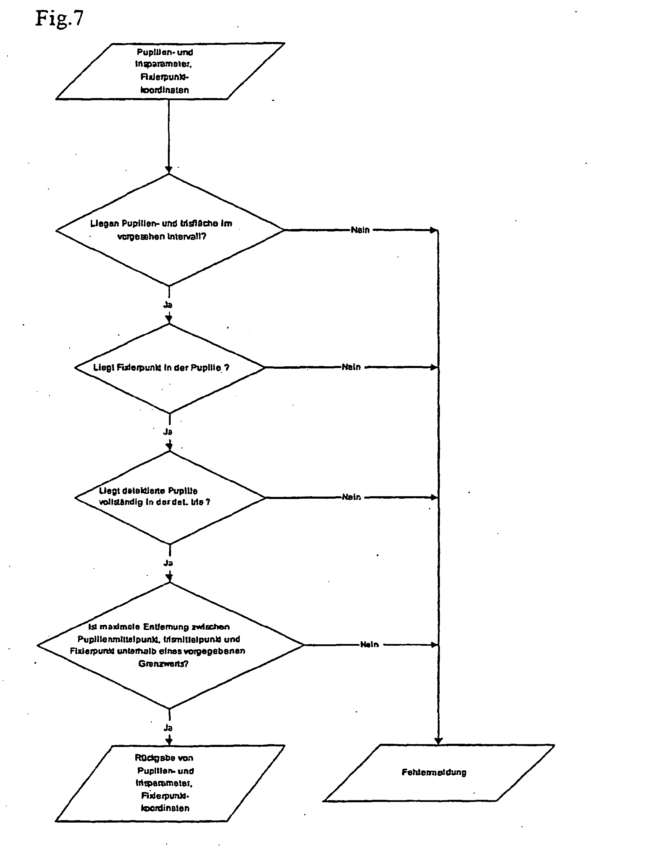

**Plausibilitätskontrolle- Fig.7**

Vor der Rückgabe der ermittelten Koordinaten an das aufrufende Programm wird eine Plausibilitätskontrolle nach Fig. 7 vorgenommen, um zu verhindern, daß eventuell unrichtig detektierte Elemente gefunden wurden. Die Abfragen beinhalten vorher bekannte Eigenschaften des untersuchten Objektes, mit welchen die ermittelten Ergebnisse in Koinzidenz stehen müssen.

**Patentansprüche**

1. Verfahren zur Ermittlung der Sehachse eines Auges (1), wobei mittels eines Fixierlichtes (3), welches sichtbans Licht aussendet, ein Durchstoßpunkt der Sehachse durch die Hornhaut ermittelt wird, wobei aus der Lage eines Bildes (12) des Fixierlichtes (3) auf der Hornhaut zu einem Pupillenmittelpunkt (14) ein Winkel ($\alpha$) zwischen Sehachse und optischer Achse des Auges (1) bestimmt wird, wobei das Auge mit kreisförmig um eine optische Geräteachse angeordneten Lichtquellen (2) beleuchtet wird, das Auge (1) in einem Beobachtungsstrahlengang telezentrisch auf eine Kamera (6) abgebildet wird, und das Fixierlicht (3) koaxial über einen Strahlteiler (4) in den Beobachtungsstrahlengang eingeblendet wird.

2. Verfahren nach Anspruch 1, wobei mittels des Winkels ($\alpha$) ein Gerät zur interferometrischen Vermessung der Teilabschnitte des menschlichen Auges (1) entlang der optischen Achse dieses Auges (1) voreingestellt wird.

**EP 2 042 078 B1**

3. Verfahren nach Anspruch 2, wobei zur Voreinstellung der Blickrichtung eines Patienten das Fixierlicht (3) nach der Ermittlung des Durchstoßpunktes und des Winkels ($\alpha$) dem Patienten unter dem ermittelten Winkel dargeboten wird.

**Claims**

1. Method for determining the visual axis of an eye (1), wherein a point at which the visual axis passes through the cornea is determined by using a fixation light (3) emitting visible light, wherein an angle ($\alpha$) between the visual axis and an optical axis of the eye (1) is determined from a position which an image (12) of the fixation light (3) on the cornea has relative to a pupil center (14), wherein the eye is illuminated from light sources (2) which are located on a circle around an optical axis of a device, the eye is imaged in telecentric manner through an observation beam path to a camera (6), and the fixation light (3) is coupled-in co-axially to the observation beam path via a beamsplitter (4).

2. Method of claim 1, wherein the angle ($\alpha$) is utilized to pre-adjust a device for interferometrically measurements of sections of the human eye (1) along an optical axis of this eye (1).

3. Method of claim 2, wherein after determining the point where the visual axis passes through the cornea and after determining the angle ($\alpha$) the fixation light is presented to a patient under the determined angle to pre-adjust the gazing direction of the patient.

**Revendications**

1. Procédé de détermination de l'axe de vision d'un oeil (1), un point de percée de l'axe de vision dans la cornée étant déterminé au moyen d'une lumière de fixation (3) qui émet de la lumière visible, un angle ($\alpha$) entre l'axe de vision et l'axe optique de l'oeil (1) étant déterminé à partir de la position d'une image (12) de la lumière de fixation (3) sur la cornée par rapport au centre (14) de la pupille, selon lequel procédé l'oeil est éclairé au moyen de sources lumineuses (2) agencées en cercle autour d'un axe optique de l'appareil, l'oeil (1) est reproduit par un système télécentrique sur une caméra (6) dans la trajectoire d'un faisceau d'observation, et la lumière de fixation (3) est injectée coaxialement dans la trajectoire du faisceau d'observation via un séparateur de faisceaux (4).

2. Procédé selon la revendication 1, selon lequel un appareil destiné à mesurer par voie interférométrique des segments partiels de l'oeil (1) humain le long de l'axe optique de cet oeil (1) est préréglé à l'appui de l'angle ($\alpha$).

3. Procédé selon la revendication 2, selon lequel, après la détermination du point de percée et de l'angle ($\alpha$), la lumière de fixation (3) est présentée au patient selon l'angle déterminé, en vue du préréglage de la direction d'observation d'un patient.

7

Fig.1

digitalisierte
Aufnahme mit
Umfeldbeleuchtung

Rauschunterdrückung

Grobdetektion der Pupille

Feindetektion der
Pupillenkante

Feindetektion der Iriskante

Dektion der Sehachse
(Fixierpunktreflex)

Plausibilitätskontrolle

Pupillen-, Iris- und
Sehachsenparameter

Fig.2

Fig.3

Fig.4

Strecke, entlang welcher Grauwerte im
gefilterten Bild abgetastet werden

GP

für Kantenort

Suchbereich

$(x_0, y_0)$

SB

S

α

K
Pupille bzw. Iris

Fig.5

Fig.6

Sonstige (nicht relevante) Binärobjekte

Dem Pupillenmittelpunkt am nächsten liegendes Binärobjekt = Fixierpunkt BF

PM

Pupillenrand

Irisrand

# Fig.7

Fig. 8 schematische Darstellung der erfindungsgemäßen Anordnung

Fig. 9 gegrabbtes Bild eines zu vermessenden Auges

Fig. 10 Vergrößerung des zentralen Teiles von Fig. 9

Fig.11 Weiß-zu-Weiß-Schablone nach Holladay-Godwin:

AE-1576 Holladay-Godwin
Cornea Guage

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4319564 A
- US 4398812 A
- EP 0550673 A
- US 5291234 A
- WO 0033729 A
- DE 19618883
- US 5231674 A
- WO 0033729 Z

**In der Beschreibung aufgeführte Nicht-Patentliteratur**

- **JACK T. HOLLADAY.** Intraocular Lens Power Calculations for the Refractive Surgeon. *Operative Techniques in Cataract and Refractive Surgery,* September 1998, vol. 1 (3), 105-117
- **GULLSTRAND ; DIEPES.** Refraktionsbestimmung. Verlag Bode, 1988
- **SAVITZKY A. ; GOLAY, M.J.E.** *Analytical Chemistry,* 1964, vol. 36, 1627-39
- **BOOKSTEIN, F.L.** Fitting conic sections to scattered data. *Computer Graphics and Image Processing,* 1979, vol. 9, 56-71
- **FITZGIBBON, A.W. ; FISHER, R.B.** A buyer's guide to conic fitting. *Proceedings of British Machine Vision Conference,* 1995